# EUROPÄISCHE PATENTSCHRIFT

(11) **EP 3 340 090 B1**
(45) Veröffentlichungstag und Bekanntmachung des Hinweises auf die Patenterteilung: **15.04.2020**
(21) Anmeldenummer: 16206397.8
(22) Anmeldetag: 22.12.2016
(51) Int. Cl.: G16H 40/63

(54) **ZUORDNUNG UNTERSCHIEDLICHER ANWENDUNGSPROGRAMME EINES MEDIZINISCHEN BILDGEBENDEN GERÄTES ZU DETEKTIERTEN ANZEIGEEINRICHTUNGEN**
ALLOCATION OF DIFFERENT APPLICATION PROGRAMS OF A MEDICAL IMAGING DEVICE TO DETECTED DISPLAY UNITS
ATTRIBUTION DE DIFFÉRENTS PROGRAMMES D'APPLICATION D'UN APPAREIL D'IMAGERIE MÉDICALE À DES ÉCRANS DÉTECTÉS

(43) Veröffentlichungstag der Anmeldung: 27.06.2018
(73) Patentinhaber: Siemens Healthcare GmbH, 91052 Erlangen (DE)
(72) Erfinder: Charrad, Chiheb, 91077 Neunkirchen A. Brand (DE); Murphy, Ivan, 91083 Baiersdorf (DE)

(56) Entgegenhaltungen:
- WO-A1-2014/162054
- US-A1- 2014 258 918
- US-A1- 2015 091 778

## Beschreibung

Die Erfindung betrifft ein Verfahren zum Betreiben eines medizinischen bildgebenden Systems mit zumindest zwei ausführbaren Anwendungsprogrammen.

In der medizinischen Bildgebung ist einer der Schlüssel für eine in unterschiedlichen Arbeitsplätzen homogen gestaltete, komfortabel effizientes Arbeiten ermöglichenden Arbeitsumgebung, eine generische Benutzeroberfläche oder Benutzerschnittstelle (User Interface) bereitzustellen, welche unabhängig von einem jeweils genutzten Anwendungsprogramm Anwendungsprogramm-Informationen der unterschiedlichen Anwendungsprogramme darstellen (oder hosten) kann. Dabei ist üblicherweise jedes Anwendungsprogramm für einen oder mehrere Arbeitsablaufläufe innerhalb eines Bereichs der medizinischen Bildgebung, beispielsweise der Magnetresonanz-Tomographie oder der Ultraschallbildgebung, entwickelt. Hier ist es oft erforderlich, an einem Arbeitsplatz unterschiedliche Anwendungsprogramme zu benutzen um die unterschiedlichen Aufgaben der täglichen Arbeitsabläufe der medizinischen Bildgebung, beispielsweise eine Bildakquise oder eine Bildnachbearbeitung, effektiv zu durchlaufen.

Typischerweise werden die erforderlichen Anwendungsprogramme von den Benutzern einzeln auf einer entsprechenden Recheneinrichtung gestartet und dann unter Rückgriff auf die Eigenschaften und Fähigkeiten eines Betriebssystems der Recheneinrichtung zwischen den verschiedenen Anwendungsprogrammen per Hand hin und her geschaltet. Diese Lösung ist nicht nur benutzerunfreundlich sondern leistet auch einer Vielzahl von Fehlern Vorschub. So kann der Benutzer bei dem manuellen Umschalten leicht einen Schritt des Arbeitsablaufes übersehen oder beispielsweise bei einem parallelen Arbeiten mit unterschiedlichen Instanzen der verschiedenen Anwendungsprogramme, welche jeweils unterschiedlichen Patienten zugeordnet sind, auch die Instanzen verwechseln, sodass es zu einem Verwechseln der Patienten beziehungsweise der Patientendaten kommen.

Um die Benutzer besser zu unterstützen, gibt es Systeme, welche die Benutzerschnittstellen verschiedener Anwendungsprogramme in ein einheitliches Benutzerinterface, einen so genannten User-Interface-(UI-)Container, integrieren. Dadurch können unterschiedliche Anwendungsprogramme verfügbare Eingabe- und/oder Ausgabeeinrichtungen nutzen, indem in die Benutzerschnittstelle Elemente eingefügt werden, mit welchen sich die jeweils gemäß dem Arbeitsablauf erforderlichen Anwendungsprogramme in den Vordergrund bringen lassen oder indem diese Elemente in einer vorgegebenen Weise den unterschiedlichen Anwendungsprogrammen jeweilige Anzeigebereiche auf einer entsprechenden Anzeigeeinrichtung zuordnen. Aber selbst dieser verbesserte Ansatz ist nicht ausreichend um die verschiedenen bildgebenden Arbeitsabläufe, welche einen Benutzer an einem Arbeitsplatz im Laufe eines Tages mit verschiedenen Patienten durchläuft, in einer angenehmen und effizienten Weise zu unterstützen.

In anderen Ansätzen wird eine Konfiguration der Anwendungsprogramme und der Ein- und Ausgabegeräte in einem Herstellungsprozess vor einer Auslieferung an einen Benutzer vorgenommen. Entsprechend werden dabei einem oder mehreren Anwendungsprogrammen fest die entsprechenden Geräte, also Eingabe- und/oder Ausgabeeinrichtungen, zugeordnet. Die Änderung einer solchen Zuordnung oder Konfiguration ist dabei über eine Anpassung der Benutzerschnittstelle möglich, welche jedoch bei einer geschulten Person vorgenommen werden muss, welche weiß welche Anforderungen von den jeweiligen Anwendungsprogrammen oder Applikationen an die entsprechenden Eingabe- und/oder Ausgabeeinrichtungen gestellt werden, da nicht jedes Anwendungsprogramm mit jeder ausgelieferten Anzeigeeinrichtungen die erwünschten medizinischen Ergebnisse liefern kann.

Der Erfindung liegt somit die Aufgabe zu Grunde, die Effizienz bei einem Betreiben eines medizinischen bildgebenden Systems mit zumindest zwei ausführbaren Anwendungsprogrammen auf für einen Nutzer komfortable Weise zu steigern und insbesondere die Fehlerhäufigkeit bei dem Betreiben des medizinischen Gerätes zu reduzieren.

Die US 2015/0091778 A1 offenbart ein Anzeigesystem um medizinische Bilder über eine Vielzahl von separaten Anzeigeeinrichtungen anzuzeigen. Dabei weist das Anzeigesystem eine Layout-Einheit auf, welche eine Information über eine gewünschte Anordnung der medizinischen Bilder beim Anzeigen bereitstellt, und eine Zuordnungseinheit um entsprechend der Anordnungsinformation eine Vielzahl von medizinischen Bildern den verschiedenen Anzeigeeinrichtungen zuzuweisen.

Die US 2014/258918 A1 offenbart ein Gerät zum Anzeigen einer medizinischen Information, bei welchem eine Entscheidungseinheit entscheidet, ob zusätzlich zu einem ersten Fenster auf einem Bildschirm mit einer vorgegebenen Größe noch ein zweites Fenster zusätzlich auf dem Bildschirm angezeigt werden soll. Ist hier für das zweite Fenster nicht genügend Platz auf dem Bildschirm vorhanden, so überprüft die Entscheidungseinheit ob das erste oder zweite Fenster in seiner Größe angepasst werden kann, sodass beide Fenster auf dem Bildschirm dargestellt werden können.

Diese Aufgabe wird durch die Gegenstände der unabhängigen Patentansprüche gelöst. Vorteilhafte Ausführungsformen ergeben sich aus unabhängigen Patentansprüchen, der Beschreibung und den Figuren.

Die Erfindung betrifft ein Verfahren zum Betreiben eines medizinischen bildgebenden Systems mit zumindest zwei ausführbaren Anwendungsprogrammen. Bei den ausführbaren Anwendungsprogrammen kann es sich beispielsweise um Computerprogramme handeln. Die Anwendungsprogramme sind dabei unabhängig voneinander, also auch alleine ausführbar. Beispielsweise dienen sie einem unterschiedlichen Zweck oder einer unterschiedlichen Funktion bei dem bildgebenden System, so beispielsweise einer Bildakquise, also einem Erfassen eines Bildes, oder einer Nachbearbeitung, also einem Bearbeiten eines erfassten Bildes.

Das Verfahren weist mehrere Verfahrensschritte auf. Ein Verfahrensschritt ist ein Detektieren zumindest einer Anzeigeeinrichtung, welche auch als eine optische Ausgabeeinrichtung bezeichnet werden kann, mit ihren jeweiligen für ein optisches Anzeigen oder Ausgeben einer Information relevanten Eigenschaften. Dabei kann auch nur eine einzige Eigenschaft detektiert werden. In diesem Fall erfolgt entsprechend ein Detektieren der Anzeigeeinrichtung mit zumindest einer relevanten Eigenschaft. Das Detektieren erfolgt durch eine Recheneinrichtung des bildgebenden Systems. Ein weiterer Verfahrensschritt ist ein Abrufen zumindest einer jeweiligen anwendungsspezifischen Anforderung der zumindest zwei Anwendungsprogramme an eine Anzeigeeinrichtung für ein optisches Anzeigen von einer jeweiligen Anwendungsprogramm-Information auf der entsprechenden Anzeigeeinrichtungen durch die Recheneinrichtung. Für jedes Anwendungsprogramm wird somit durch die Recheneinrichtung eine anwendungsspezifische Anforderung abgerufen. Die abgerufenen Anforderungen), wobei die abgerufene Anforderung einen oder mehrere der folgenden Parameter der jeweiligen Anzeigeeinrichtung (3a, 3b, 3c) umfasst: eine erforderliche oder empfohlene Bildauflösung, eine erforderliche oder empfohlene Bildwiederholrate, einen erforderlichen oder empfohlenen Farbraum, eine Kalibrierungsinformation, eine empfohlene oder erforderliche Größe einer Anzeigefläche (7a, 7b), eine empfohlene oder erforderliche Ausrichtung, eine Anordnungsinformation, eine Gattungsinformation, ob jeweils ein Bildschirm oder ein berührungsempfindlicher Bildschirm oder ein 3-D-Bildschirm oder ein Projektor oder eine Datenbrille empfohlen oder erforderlich ist Die anwendungsspezifischen Anforderungen können beispielsweise in Form eines Anforderungsprofils des jeweiligen Anwendungsprogramms abgerufen werden. Die Anforderung kann auch die Information enthalten, dass keine (besonders beachtenswerte) Anforderung durch das Anwendungsprogramm an die Anzeigeeinrichtung gestellt wird. Die Anforderung kann auch eine Mindestanforderung und/oder eine Optimalanforderung umfassen. Die Anforderung kann dabei im bildgebenden System hinterlegt sein. Die Anforderung kann für ein Anwendungsprogramm auch anhand der Kategorie oder des Verwendungszweckes des Anwendungsprogramms abgerufen oder durch die Recheneinrichtung bestimmt werden. So benötigen beispielsweise Anwendungsprogramme, welche für ein Mammographiesystem als bildgebendes System ausgebildet sind, typischerweise zwei hochauflösende Monitore im Hochformat, wohingegen Anwendungen für ein einfaches Computertomographiesystem als bildgebendes medizinisches System typischerweise nur einen einzigen Monitor im Querformat, gegebenenfalls auch einen berührungsempfindlichen Monitor, benötigen. Anwendungsprogramme für ein hochwertiges Magnetresonanz-Tomografiesystem benötigen wiederum im Allgemeinen für einen optimalen und effektiven Arbeitsablauf zwei Monitore im Querformat. Zusätzlich kann ein Benutzer entsprechend individuellen Bedürfnissen auch noch einen zusätzlichen Monitor zu dem jeweiligen System hinzuzufügen, beispielsweise für ein parallel zu anderen Anwendungsprogrammen laufendes radiologisches Informationssystem (radiology information system, RIS) oder für eine Arbeits- oder To-do-Liste, welche beispielsweise einen Arbeitsablauf oder Workflow begleiten. Auch dies kann beispielsweise in einem Anforderungsprofil als anwendungsspezifische Anforderung hinterlegt sein.

Ein weiterer Verfahrensschritt ist ein Vergleichen der jeweils für die zumindest eine Anzeigeeinrichtung detektierten Eigenschaft(en) und der abgerufenen anwendungsspezifischen Anforderung(en) durch die Recheneinrichtung. Dabei betreffen Eigenschaften und Anforderungen naturgemäß jeweils die gleichen Parameter. Es kann somit beispielsweise eine erforderliche Mindestauflösung für ein Mammografie-Anwendungsprogramm mit einer verfügbaren maximalen Auflösung einer detektierten Anzeigeeinrichtung verglichen werden.

Ein nächster Verfahrensschritt ist dann in Abhängigkeit eines Ergebnisses des Vergleiches ein jeweiliges Zuordnen der Anwendungsprogramme zu der zumindest einen Anzeigeeinrichtung oder ein Aktivieren einer Zuordnungsmöglichkeit für einen Benutzer durch die Recheneinrichtung. Dabei kann beispielsweise dem Benutzer durch die zumindest eine detektierte Anzeigeeinrichtung eine Zuordnungsmöglichkeit angezeigt werden. Es kann so beispielsweise einem Anwendungsprogramm automatisch die Anzeigeeinrichtung zugeordnet werden, welche dem entsprechenden Anforderungsprofil, das heißt der anwendungsspezifischen Anforderung genügt oder die besten medizinischen Ergebnisse liefern kann. Es kann dabei beispielsweise auch ein jeweiliger Anzeigebereich oder Bildbereich der zumindest einen Anzeigeeinrichtung dem oder den jeweiligen Anwendungsprogrammen zugeordnet werden.

Als weiterer Verfahrensschritt kann auch ein Anzeigen einer jeweiligen Anwendungsprogramm-Information des Anwendungsprogramms auf der zumindest einen dem jeweiligen Anwendungsprogramm zugeordneten Anzeigeeinrichtung erfolgen.

Das hat den Vorteil, dass die Benutzerschnittstelle für die Anwendungsprogramme jeweils optimal an die zumindest eine Anzeigeeinrichtung und deren jeweilige technischen Möglichkeiten angepasst werden kann. Dabei benötigen die Anwendungsprogramme selber keine Information über die Anzeigeeinrichtung oder Anzeigeeinrichtungen, da die Koordination des optischen Anzeigens der Anwendungsprogramm-Informationen der jeweiligen Anwendungsprogramme, also das "Anzeigenmanagement", von der Recheneinrichtung übernommen wird. Somit kann sichergestellt werden, dass die Benutzerschnittstelle unabhängig von einer zu einem Produktionszeitpunkt des Systems gegebenen Konfiguration des Systems den entsprechenden Anforderungen der Anwendungsprogramme und des jeweiligen medizinischen Szenarios genügt. Die Anwendungsprogramme müssen somit die Hardware, das heißt die Anzeigeeinrichtungen nicht kennen, das System beziehungsweise die Recheneinrichtung hingegen weiß aufgrund der abgerufenen anwendungsspezifischen Anforderungen, dass beispielsweise ein Mammografie-Anwendungsprogramm einen hochauflösenden Bildschirm oder eine hochauflösende Anzeigeeinrichtung benötigt, und kann diesen nutzen, wenn er vorhanden ist und detektiert wird. So keine hochauflösende Anzeigeeinrichtung detektiert wird, oder möglicherweise das System mehrere geeignete Anzeigeeinrichtungen detektiert oder aus sonstigen Gründen nicht entscheiden kann, welche Anzeigeeinrichtung dem entsprechenden Anwendungsprogramm zugeordnet werden soll, kann das System den Benutzer über das Aktivieren der Zuordnungsmöglichkeit für den Benutzer fragen und dieser entscheiden. Dabei kann die entsprechende Nutzereingabe insbesondere abgespeichert und als neue anwendungsspezifische Anforderung beispielsweise eine fabrikseitige oder herstellerseitige Einstellung überschreiben oder ergänzen und bei einem künftigen Durchlaufen des Verfahrens als anwendungsspezifische Anforderung genutzt werden. Somit ist ein mit dem beschriebenen Verfahren arbeitendes bildgebendes System in vielen verschiedenen Anwendungsszenarios flexibel anwendbar. Das System kann mit den Anwendungsprogrammen mit einem geringen Aufwand auf unterschiedlichen Hardwareimplementierungen implementiert werden, da die jeweiligen Anwendungsprogramme nicht an die möglichen Hardwarekonfiguration des Systems angepasst werden müssen. So kann beispielsweise eine Plattform für das bildgebende medizinische System bereitgestellt werden, welche dann von unterschiedlichen Seiten, beispielsweise einem Endnutzer oder aber einem Weiterverkäufer mit jeweils unterschiedlicher Hardware an individuelle Vorstellungen angepasst und mit den gleichen Anwendungsprogrammen betrieben werden kann.

In einer vorteilhaften Ausführungsform ist dabei vorgesehen, dass das Zuordnen ein Zuteilen von unterschiedlichen Anzeigebereichen der zumindest einen Anzeigeeinrichtung, also einer oder mehrerer Anzeigeeinrichtungen, zu den jeweiligen Anwendungsprogrammen umfasst. Es können somit unterschiedliche Anzeigebereiche einer einzigen Anzeigeeinrichtung den unterschiedlichen Anwendungsprogrammen zugeordnet werden, oder aber unterschiedliche Anzeigebereiche verschiedener Anzeigeeinrichtungen. Insbesondere können die jeweils in einer Anzeigeeinrichtung verfügbaren Anzeigebereiche vollständig einem jeweiligen Anwendungsprogramm zugeteilt werden, so dass auf einer Anzeigeeinrichtung jeweils eine Anwendungsprogramm-Information nur eines einzigen Anwendungsprogrammes angezeigt wird.

Das hat den Vorteil, dass eine besonders übersichtliche Arbeitsumgebung geschaffen wird. Beispielsweise können so die Anwendungsprogramme jeweils unterschiedlichen Fenstern auf einer Anzeigeeinrichtung zugeordnet werden, oder aber einem Vollbild auf einer Anzeigeeinrichtung. Dies kann beispielsweise in der Art erfolgen, in welcher bei einem Bedienen eines Computers mit einem Webbrowser manuell unterschiedliche Tabs eines Webbrowsers in Fenster aufgelöst werden können, welcher dann frei positionierbar auf einer oder mehreren Anzeigeeinrichtungen verteilt werden können.

Das hat den Vorteil, dass eine besonders flexible Darstellung erreicht wird. Das beschriebene Verfahren erlaubt ein automatisches Berechnen und entsprechendes Skalieren der anzuzeigenden Anwendungsprogramm-Information auf die zugeteilten Anzeigebereiche beziehungsweise die zugeordnete Anzeigeeinrichtung. Da sich die unterschiedlichen Anzeigeeinrichtungen signifikant in größeren Auflösungsvermögen sowie in weiteren Eigenschaften unterscheiden können, kann so ein Anzeigepotenzial des medizinischen Systems besser genutzt werden.

In einer weiteren vorteilhaften Ausdrucksform ist vorgesehen, dass mehrere Anzeigeeinrichtungen detektiert werden. Insbesondere unterscheiden sich von den mehreren Anzeigeeinrichtungen dabei zumindest zwei Anzeigeeinrichtungen in ihren für das optische Anzeigen relevanten Eigenschaften.

Gerade bei mehreren Anzeigeeinrichtungen führt ein Detektieren und entsprechendes anwendungsspezifisches Zuordnen der Anzeigeeinrichtungen zu den Anwendungsprogrammen anhand ihrer Eigenschaften zu einer besonders großen Steigerung der Effizienz und des Komforts. Dies gilt gerade für Anzeigeeinrichtungen mit unterschiedlichen Eigenschaften.

In einer weiteren vorteilhaften Ausführungsform ist vorgesehen, dass die zumindest eine detektierte Anzeigeeinrichtung, insbesondere eine von oder bevorzugt alle von mehreren detektierten Anzeigeeinrichtungen, jeweils ein Bildschirm oder ein berührungsempfindlicher Bildschirm oder ein 3-D Bildschirm zur dreidimensionalen Darstellung von Bildern oder ein Projektor oder eine Datenbrille, bevorzugt eine Datenbrille für eine virtuelle und/oder erweiterte Realität oder eine sonstige Anzeigeeinrichtungen ist.

Für die genannten Anzeigeeinrichtungen ist das beschriebene Verfahren besonders vorteilhaft, da diese teilweise stark unterschiedliche Eigenschaften aufweisen und jedoch sämtlich in einem medizinischen Szenario mit einem bildgebenden System zum Einsatz kommen können. Das beschriebene Verfahren ermöglicht hier auf besonders einfache Weise ein Ausnutzen der unterschiedlichen Eigenschaften für ein effizientes Arbeiten mit dem System.

In einer anderen vorteilhaften Ausführungsform ist vorgesehen, dass die detektierte Eigenschaft und entsprechend die abgerufenen Anforderung einen oder mehreren der folgenden Parameter der jeweiligen Anzeigeeinrichtungen beziehungsweise des jeweiligen Anwendungsprogrammes betrifft oder umfasst: eine darstellbare beziehungsweise erforderliche oder empfohlene Bildauflösung, eine darstellbare beziehungsweise erforderliche oder empfohlene Bildwiederholrate, einen darstellbaren beziehungsweise erforderlichen oder empfohlenen Farbraum, eine Kalibrierungsinformation, eine vorhandene beziehungsweise empfohlene oder erforderliche Größe einer Anzeigefläche, eine vorhandene beziehungsweise empfohlene oder erforderliche Ausrichtung, das heißt beispielsweise eine horizontale oder eine vertikale Ausrichtung oder Haupterstreckung, der Anzeigefläche, eine Anordnungsinformation, beispielsweise betreffend der relativen Anordnung einer Anzeigeeinrichtung zu einer weiteren Anzeigeeinrichtung der mehreren Anzeigeeinrichtungen, eine Gattungsinformation, aus welcher insbesondere hervorgeht, ob die jeweilige Anzeigeeinrichtung ein gewöhnlicher Bildschirm oder ein berührungsempfindlicher Bildschirm oder ein 3-D-Bildschirm oder ein Projektor oder eine Datenbrille, bevorzugt eine Datenbrille für eine virtuelle und/oder erweiterte Realität, oder eine sonstige Einrichtung ist, beziehungsweise ob jeweils ein Bildschirm oder ein berührungsempfindlicher Bildschirm oder ein 3-D-Bildschirm oder ein Projektor oder eine Datenbrille empfohlen oder erforderlich ist.

Die genannten Eigenschaften haben den Vorteil, dass sich anhand ihrer die jeweilige Eignung der Anzeigeeinrichtung für die Anwendungsprogramme im medizinischen Bereich besonders gut feststellen lässt, sodass die Effizienz des Verfahrens und die Nutzerfreundlichkeit weiter verbessert wird.

In einer bevorzugten Ausführungsform ist vorgesehen, dass das Verfahren automatisch bei einem Startvorgang oder beim Hochfahren des medizinischen bildgebenden Systems durchgeführt wird. Beispielsweise kann so bei einem Hersteller eine Standardkonfiguration für die Anwendungsprogramme und mehrere, beispielsweise zumindest zwei, Bildschirme oder Monitore vorgegeben werden, bei welchen zwei Anzeigeeinrichtungen unterschiedlichen Anwendungsprogrammen zugeordnet werden.

Das System kann dabei mit entsprechend zwei Anzeigeeinrichtungen ausgeliefert und in einem 2-Monitor-Modus betrieben werden. Wird nun beispielsweise durch Dritte, sei es einen Benutzer oder einen Wiederverkäufer, nun eine dritte Anzeigeeinrichtung angeschlossen, so wird diese von dem System automatisch erkannt und, wenn dies in dem Anforderungsprofil oder in der anwendungsspezifischen Anforderungen hinterlegt ist, beispielsweise auf einen 3-Monitor-Modus für das System mit den drei Anzeigeeinrichtungen umgeschaltet. Alternativ kann zum Beispiel so auch wenn eine Anzeigeeinrichtungen ausfällt dieses detektiert werden. Dann wird nicht etwa wie sonst üblich das Anwendungsprogramm, welches dem defekten Bildschirm zugeordnet ist auf diesem dargestellt werden soll, einfach für einen Benutzer unsichtbar bleiben, sondern dem Benutzer ermöglicht, dem Anwendungsprogramm eine andere Anzeigevorrichtung zuzuordnen.

Das hat den Vorteil, dass unabhängig von einem Zustand des bildgebenden Systems bei einer Auslieferung stets eine optimierte Zuordnung von Anwendungsprogrammen und Anzeigeeinrichtungen realisiert ist. Überdies nutz das System besonders flexibel und anwendungsfreundlich vorhandene Kapazitäten zum Anzeigen von Informationen.

In einer weiteren vorteilhaften Ausführungsform ist vorgesehen, dass die Anwendungsprogramme zu unterschiedlichen Kategorien gehören, insbesondere jeweils zu einer der Kategorien Planung, wie beispielsweise einer Arbeitsliste, Bildakquise wie beispielsweise ein Scannen eines Patienten oder Aufnehmen eines Bildes, Nachbearbeitung wie beispielsweise ein PostProcessing von aufgenommenen Bilddaten oder Darstellung wie beispielsweise ein Präsentieren von Bildergebnissen an den Benutzer des Systems oder weitere Personen.

Das hat den Vorteil, dass generisch für unterschiedliche Anwendungsprogramme jeweilige anwendungsspezifische Anforderungen abgerufen werden können, ohne dass das jeweilige Anwendungsprogramm genau identifiziert ist. Dies erhöht die Flexibilität des Systems weiterhin.

In einer weiteren vorteilhaften Ausführungsform vorgesehen, dass die Anwendungsprogramme für die Verwendung in einem Ultraschallsystem und/oder in einem Mammographiesystem und/oder in einem Computertomographiesystem und/oder in einem Magnetresonanzsystem geeignet sind.

In diesem Fall hat das Verfahren den Vorteil, dass das System für unterschiedliche Verwendungszwecke genutzt werden kann und sich dabei flexibel an die jeweiligen Bedingungen anpassen kann, beziehungsweise für die jeweiligen Bedingungen in den unterschiedlichen Bereichen der Bildgebung so angepasst werden kann, dass es sich über das hier beschriebene Verfahren an die jeweilige Verwendung anpasst.

In einer weiteren vorteilhaften Ausführungsform ist vorgesehen, dass für die Anwendungsprogramme in der Recheneinrichtung zumindest ein Arbeitsablauf, ein so genannter Workflow, vorgegeben wird, durch welchen ein zeitlicher Ablauf für das Aktivieren und/oder Durchlaufen der Anwendungsprogramme, also beispielsweise ein gleichzeitig oder hintereinander erfolgendes Durchlaufen und/oder Aktivieren der Anwendungsprogramme, und ein Anzeigen einer jeweiligen Anwendungsprogramm-Information der jeweiligen Anwendungsprogramme auf der dem jeweiligen Anwendungsprogramm zugeordneten Anzeigeeinrichtung vorgegeben wird.

Der Arbeitsablauf kann dabei beispielsweise vorgegeben werden, in dem ein automatisch gestartetes Rahmenprogramm, eine Masteranwendung oder Masterapplikation, die Anwendungsprogramme des Systems gemäß einem vorgegebenen Schema aktiviert oder dem Nutzer anbietet die Anwendungsprogramme zu aktivieren. Insbesondere können die Anwendungsprogramme dabei nur über das Rahmenprogramm aktivierbar sein. Auf der zugeordneten Anzeigeeinrichtung kann dabei beispielsweise durch das Rahmenprogramm dem jeweiligen Anwendungsprogramm oder den Anwendungsprogrammen ein entsprechender Anzeigebereich zugeteilt werden. Dabei können für einen jeweiligen Arbeitsablauf jeweils für unterschiedliche Anwendungsprogramme, beispielsweise unterschiedliche Kategorien von Anwendungsprogrammen, jeweilige anwendungsspezifische Anforderungen vorgesehen sein.

Die anwendungsspezifischen Anforderungen können somit auch von dem Arbeitsablauf abhängen. So kann beispielsweise eine gewisse Anordnung oder Topologie der Anzeigeeinrichtungen an dem Arbeitsplatz für einen jeweiligen Arbeitsablauf hinterlegt sein. Für einen anderen Arbeitsablauf kann dabei eine andere Anordnung hinterlegt sein. Dies erlaubt eine nutzerspezifische Anpassung des Arbeitsablaufs mit entsprechenden unterschiedlichen Anforderungen für die Anwendungsprogramme. Dabei können bestimmte Anforderungen für die Anwendungsprogramme auch in einer von einem Nutzer unveränderbaren Weise vorgegeben sein, um einer Fehlbenutzung vorzubeugen. Gleichzeitig kann das bildgebende System so individualisiert und an individuelle Bedürfnisse angepasst werden. Somit können die jeweiligen Anwendungsprogramme in Abhängigkeit des vorgegebenen Arbeitsablaufes in einem vorgegebenen Kontext aktiviert werden und den unterschiedlichen Anzeigeeinrichtungen zugeordnet werden.

Entsprechend ist in einer weiteren vorteilhaften Ausführungsform vorgesehen, dass der Arbeitsablauf und damit insbesondere auch die anwendungsprogrammspezifischen Anforderungen durch einen Nutzer angepasst werden kann und der angepasste Arbeitsablauf in der Recheneinrichtung hinterlegt werden kann.

Das Rahmenprogramm kann somit ein sogenanntes Framework für die anderen Anwendungsprogramme bereitstellen, insbesondere inklusive spezifischer Benutzerschnittstellenelemente um zwischen den verschiedenen Anwendungsprogrammen hin und her zu schalten. Die entsprechenden Anwendungsprogramme welche dem Rahmenprogramm zugeordnet sind, können dabei insbesondere kein eigenes Framework aufweisen, das heißt sie können entweder automatisch, beispielsweise ausgelöst durch ein Ereignis eines anderen Anwendungsprogrammes und/oder als Teil des Arbeitsablaufs automatisch oder halbautomatisch ausgelöst oder aktiviert werden, beispielsweise durch ein konfigurierbares oder dynamisch hinzugefügtes Bedienelement der Nutzerschnittstelle, zum Beispiel einen Knopf der durch das Rahmenprogramm dargestellt wird.

Das hat den Vorteil, dass die Anwendungsprogramme besonders effizient, effektiv, fehlersicher und benutzerfreundlich den entsprechenden Anzeigeeinrichtungen zugewiesen werden.

In einer weiteren vorteilhaften Ausführungsform ist vorgesehen, dass durch den Arbeitsablauf zumindest eine jeweilige Instanz der unterschiedlichen Anwendungsprogramme aktiviert wird, welche der Behandlung zumindest eines jeweiligen Patienten durch das medizinische bildgebende System dient, und Instanzen der unterschiedlichen Anwendungsprogramme, welche der Behandlung des selben Patienten dienen, als solche markiert und/oder gleichzeitig dargestellt und/oder auf der benachbarten Anzeigebereichen und/oder nebeneinander angeordneten Anzeigeeinrichtungen angezeigt werden.

Das hat den Vorteil, dass durch den Benutzer sofort auf einfache Weise erkannt werden kann, welche Instanzen beziehungsweise welche Anwendungsprogramm-Information zu dem jeweils gleichen Patienten gehört. Damit werden auf komfortable Weise eine Fehlbedienung oder Irrtümer bei der Behandlung des Patienten vermieden.

Die Erfindung betrifft auch ein medizinisches bildgebendes System mit zumindest zwei ausführbaren Anwendungsprogrammen, mit einer Recheneinrichtung, welche ausgebildet ist, a) zumindest eine Anzeigeeinrichtung mit ihren für ein optisches Anzeigen relevanten Eigenschaften zu detektieren, b) zumindest eine jeweilige anwendungsspezifische Anforderung der zumindest zwei Anwendungsprogramme an eine Anzeigeeinrichtung für ein optisches Anzeigen von einer jeweiligen Anwendungsprogramm-Information der jeweiligen Anwendungsprogramme auf einer Anzeigeeinrichtung abzurufen, c) die jeweils für die zumindest eine Anzeigeeinrichtung detektierten Eigenschaften mit der abgerufenen Anforderung zu vergleichen sowie d) in Abhängigkeit eines Ergebnisses des Vergleichens entweder die Anwendungsprogramme jeweils zu der zumindest einen Anzeigeeinrichtungen zuzuordnen oder eine Zuordnungsmöglichkeit für einen Benutzer zu aktivieren. Bei mehreren Anzeigeeinrichtungen kann die Recheneinrichtung ausgelegt sein, jedem Anwendungsprogramm jeweils eine andere Anzeigeeinrichtung zuzuordnen.

Vorteile und vorteilhafte Ausführungsformen des medizinischen bildgebenden Systems oder Gerätes entsprechen hier Vorteilen und vorteilhaften Ausführungsformen des beschriebenen Verfahrens zum Betreiben eines medizinischen bildgebenden Systems.

Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Ausführungsbeispiele der Erfindung werden nachfolgend anhand schematischer Zeichnung näher erläutert. Dabei zeigen:
- FIG 1: eine erste beispielhafte Ausführungsform eines medizinischen bildgebenden Systems; und
- FIG 2: eine zweite beispielhafte Ausführungsform eines medizinischen bildgebenden Systems.

In den Figuren werden gleiche oder funktionsgleiche Elemente mit den gleichen Bezugszeichen versehen.

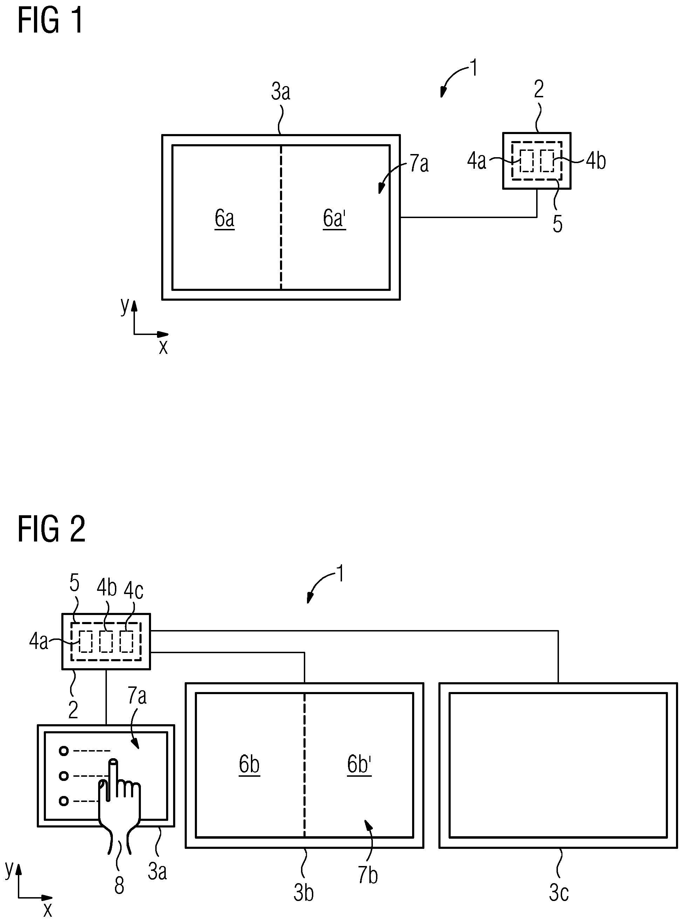

In FIG 1 ist eine beispielhafte Ausführungsform eines medizinischen bildgebenden Systems dargestellt. Das medizinische bildgebende System 1 umfasst hier vorliegend eine Recheneinrichtung 2 sowie eine Anzeigeeinrichtung 3a. Die Recheneinrichtung 2 ist dabei ausgebildet, die Anzeigeeinrichtung 3a mit ihren für ein optisches Anzeigen relevanten Eigenschaften zu detektieren.

Ferner sind vorliegend in der Recheneinrichtung 2 zwei Anwendungsprogramme 4a, 4b vorhanden, welche über ein Rahmenprogramm 5 aktiviert werden können. Die Recheneinrichtung 2 ist nun des Weiteren ausgebildet, zumindest eine jeweilige anwendungsspezifische Anforderung der zumindest zwei Anwendungsprogramme 4a, 4b an allgemein eine, vorliegend die Anzeigeeinrichtungen 3a für ein optisches Anzeigen von einer jeweiligen Anwendungsprogramm-Information auf allgemein einer, vorliegend der Anzeigeeinrichtung 3a abzurufen. So kann beispielsweise für die jeweiligen Anwendungsprogramme 4a, 4b eine Mindestgröße oder eine Orientierung einer Anzeigefläche 7a der Anzeigeeinrichtung 3a vorgesehen sein. Die Recheneinrichtung 2 ist des Weiteren ausgebildet, die jeweils für die Anzeigeeinrichtung 3a detektierten Eigenschaften, beispielsweise eine Bildschirmgröße, und die abgerufene Anforderung für die beiden Anwendungsprogramme 4a, 4b zu vergleichen.

Beispielsweise können so die beiden Anwendungsprogramme 4a, 4b jeweils eine gewisse Mindestgröße einer Darstellung auf der Anzeigeeinrichtung 3a vorsehen. Die Recheneinrichtung 2 ist nun des Weiteren ausgebildet, in Abhängigkeit eines Ergebnisses des Vergleichens entweder die Anwendungsprogramme 4a, 4b jeweils der Anzeigeeinrichtungen 3a zuzuordnen, oder eine Zuordnungsmöglichkeit für einen Benutzer zu aktivieren. Vorliegend kann so ein jeweiliger Anzeigebereich 6a, 6a' auf der Anzeigefläche 7a der Anzeigeeinrichtung 3a den beiden Programmen 4a, 4b zugeteilt werden. Entspricht der jeweilige Bereich nun beispielsweise in seiner Größe den Anforderungen der Anwendungsprogramme 4a, 4b, so wird im gezeigten Beispiel eine jeweilige Anwendungsprogramm-Information des ersten Anwendungsprogramms 4a in dem ersten Anzeigebereich 6a dargestellt und des zweiten Anwendungsprogramms 4b in dem zweiten Anzeigebereich 6a' der Anzeigeeinrichtung 3a. Ist nun beispielsweise die Anzeigeeinrichtung 3a in ihrer Größe nicht groß genug um den Anforderungen der beiden Anwendungsprogramme 4a ,4b zu genügen, so kann entsprechend der Benutzer aufgefordert werden entweder eine zusätzlich Anzeigeeinrichtung bereitzustellen oder die Anzeigebereiche 6a, 6b per Hand zuzuteilen, beispielsweise die Anzeigefläche 7a seinen Bedürfnissen entsprechend in die Anzeigebereiche 6a, 6b aufzuteilen und so den beiden Anwendungsprogrammen 4a, 4b unterschiedlich große Bildbereiche zuzuordnen.

In FIG 2 ist eine weitere beispielhafte Ausführungsform eines medizinischen bildgebenden Systems dargestellt. In diesem Beispiel umfasst das medizinische bildgebende System 1 drei Anzeigeeinrichtungen 3a, 3b, 3c, wovon die erste Anzeigeeinrichtung 3a als berührungsempfindliche Anzeigeeinrichtung und somit zugleich auch als Eingabeeinrichtung ausgeführt ist. Die weiteren beiden Anzeigeeinrichtungen 3b, 3c sind dabei vorliegend Bildschirme, welche sich im Querformat hauptsächlich in der x-Richtung erstrecken. Die Recheneinrichtung 2 ist dabei wie in FIG 1 mit mehreren, vorliegend drei Anwendungsprogrammen 4a, 4b, 4c versehen, welche ebenfalls durch das Rahmenprogramm 5 aktiviert werden.

Vorliegend sind dabei die Anwendungsprogramme 4a, 4b, 4c jeweilige unterschiedlichen Kategorien zugeordnet, nämlich das erste Programm 4a einer Planung, das zweite Anwendungsprogramm 4b einer Bildakquise und das dritte Anwendungsprogramm 4c eine Nachbearbeitung. Entsprechend jeweils für die unterschiedlichen Kategorien hinterlegter Anforderungen detektiert nun die Recheneinrichtung 2 die Anzeigeeinrichtungen 3a bis 3c mit ihren jeweils für das optische Anzeigen oder Bedienen relevanten Eigenschaften.

Nachdem hier eine berührungsempfindliche Anzeigeeinrichtung als erste Anzeigeeinrichtung 3a erkannt wird, wird diese durch die Recheneinrichtung 2 beispielsweise dem ersten Anwendungsprogramm 4a zugeordnet. Hierdurch kann ein Benutzer 8 entsprechend intuitiv auf der Anzeigefläche 7a der ersten Anzeigeeinrichtung 3a auf intuitive Weise eine Arbeitsliste bearbeiten, beispielsweise markieren oder abhaken. Die beiden weiteren Anzeigeeinrichtungen 3b, 3c sind vorliegend gewöhnliche Anzeigeeinrichtungen und somit vorliegend gleichermaßen für eine Bildgebung oder Nachbearbeitung geeignet. Entsprechend wird hier der zweiten und dritten Anzeigeeinrichtung 3b, 3c jeweils das zweite und dritte Anwendungsprogramm 4b, 4c zugeordnet. Dies alles erfolgt in diesem Beispiel automatisch.

Zu den Vorzügen des beschriebenen Verfahrens gehört es nun, dass beispielsweise wenn die dritte Anzeigeeinrichtungen 3c defekt ist, dies durch die Recheneinrichtung 2 erkannt wird und somit das dritte Anwendungsprogramm 4c auch der zweiten Anzeigeeinrichtungen 3b zugeordnet werden kann oder wird. Beispielsweise können so jeweilige Anzeigebereiche 6b, 6b' der Anzeigefläche 7b der zweiten Anzeigeeinrichtung 6b den unterschiedlichen Anwendungsprogrammen 4b und 4c zugeordnet werden. Dies erfolgt im gezeigten Beispiel nur automatisch, wenn wie in dem in FIG 1 dargestellten Beispiel dies auch gemäß der abgerufenen anwendungsspezifischen Anforderungen oder eines aktiven Benutzerwunsches sinnvoll ist. Beispielsweise kann dies realisiert werden, in dem bei einem Hochfahren automatisch die Anzeigeeinrichtungen 3a, 3b, 3c detektiert werden und deren Eigenschaften mit den anwendungsspezifischen Anforderungen verglichen werden.

Auf diese Weise kann auch eine erhöhte Flexibilität erreicht werden, da die dritte Anzeigeeinrichtung 3c nicht nur defekt sein kann sondern entfernt oder hinzugefügt werden kann, wie dies einem jeweiligen Nutzer sinnvoll erscheint. So kann beispielsweise das bildgebende System 1 auch mit zwei Anzeigeeinrichtungen 3a, 3b ausgeliefert werden und sodann im späteren Verlauf die zusätzliche Anzeigeeinrichtung 3c hinzugefügt werden, welche dann automatisch erkannt und in für die Anwendungsprogramme 4a bis 4c in optimaler Weise in dem bildgebenden System 1 genutzt wird.

Selbstverständlich können die detektierten Eigenschaften sowie die entsprechenden anwendungsspezifischen Anforderungen nicht nur die hier beschriebenen Parameter der Größe der Anzeigefläche 7a, 7b oder der Parameter einer Gattungsinformation, nämlich ob das Anzeigeelement 3a, 3b, 3c berührungsempfindlich ist oder nicht, umfassen, sondern auch viele andere Parameter, wie beispielsweise Bildauflösung, Bildwiederholrate, Farbraum, Kalibrierungsinformation, Ausrichtung der Anzeigefläche 7a, 7b, Anordnungsinformation, nach welcher beispielsweise der zweiten Anzeigeeinrichtung 3b vorliegend eine mittlere Anzeigeeinrichtung ist umfassen.

## Patentansprüche

1. Verfahren zum Betreiben eines medizinischen bildgebenden Systems (1) mit zumindest zwei unabhängig voneinander ausführbaren Anwendungsprogrammen (4a, 4b, 4c), mit den Verfahrensschritten:
a) Detektieren zumindest einer Anzeigeeinrichtung (3a, 3b, 3c) mit ihren für ein optisches Anzeigen relevanten Eigenschaften durch eine Recheneinrichtung (2) des bildgebenden Systems (1);
b) Abrufen zumindest einer jeweiligen anwendungsspezifischen Anforderung der zumindest zwei unabhängig voneinander ausführbaren Anwendungsprogramme (4a, 4b, 4c) an eine Anzeigeeinrichtung (3a, 3b, 3c) für ein optisches Anzeigen von einer jeweiligen Anwendungsprogramm-Information auf einer Anzeigeeinrichtung (3a, 3b, 3c) durch die Recheneinrichtung (2), wobei die abgerufene Anforderung einen oder mehrere der folgenden Parameter der jeweiligen Anzeigeeinrichtung (3a, 3b, 3c) umfasst: eine erforderliche oder empfohlene Bildauflösung, eine erforderliche oder empfohlene Bildwiederholrate, einen erforderlichen oder empfohlenen Farbraum, eine Kalibrierungsinformation, eine empfohlene oder erforderliche Größe einer Anzeigefläche (7a, 7b), eine empfohlene oder erforderliche Ausrichtung, eine Anordnungsinformation, eine Gattungsinformation, ob jeweils ein Bildschirm oder ein berührungsempfindlicher Bildschirm oder ein 3-D-Bildschirm oder ein Projektor oder eine Datenbrille empfohlen oder erforderlich ist;
c) Vergleichen der jeweils für die zumindest eine Anzeigeeinrichtung (3a, 3b, 3c) detektierten Eigenschaften und der abgerufenen Anforderung;
d) in Abhängigkeit eines Ergebnisses des Vergleichens entweder ein jeweiliges Zuordnen der unabhängig voneinander ausführbaren Anwendungsprogramme (4a, 4b, 4c) zu der zumindest einen Anzeigeeinrichtung (3a, 3b, 3c) oder ein Aktivieren einer Zuordnungsmöglichkeit für einen Benutzer (8) durch die Recheneinrichtung (2).

2. Verfahren nach Anspruch 1,
**dadurch gekennzeichnet, dass**
das Zuordnen ein Zuteilen von unterschiedlichen Anzeigebereichen (6a, 6a', 6b, 6b') der zumindest einen Anzeigeeinrichtung (3a, 3b, 3c) zu den jeweiligen Anwendungsprogrammen (4a, 4b, 4c) umfasst.

3. Verfahren nach einem der vorhergehenden Ansprüche,
**dadurch gekennzeichnet, dass**
mehrere Anzeigeeinrichtungen (3a, 3b, 3c) detektiert werden, von denen insbesondere zumindest zwei Anzeigeeinrichtungen (3a, 3b, 3c) sich in ihren für das optische Anzeigen relevanten Eigenschaften unterscheiden.

4. Verfahren nach einem der vorhergehenden Ansprüche,
**dadurch gekennzeichnet, dass**
die zumindest eine detektierte Anzeigeeinrichtung (3a, 3b, 3c), insbesondere eine der mehreren detektierten Anzeigeeinrichtungen (3a, 3b, 3c), ein Bildschirm oder ein berührungsempfindlicher Bildschirm oder ein 3D-Bildschirm oder ein Projektor oder eine Datenbrille, bevorzugt eine Datenbrille für eine virtuelle und/oder erweiterte Realität ist.

5. Verfahren nach einem der vorhergehenden Ansprüche,
**dadurch gekennzeichnet, dass**
die detektierte Eigenschaft einen oder mehrere der folgenden Parameter der jeweiligen Anzeigeeinrichtung (3a, 3b, 3c) umfasst: eine darstellbare Bildauflösung, eine darstellbare Bildwiederholrate, einen darstellbaren Farbraum, eine Kalibrierungsinformation, eine Größe einer Anzeigefläche (7a, 7b), eine Ausrichtung der Anzeigefläche (7a, 7b), eine Anordnungsinformation, eine Gattungsinformation, aus welcher insbesondere hervorgeht, ob die jeweilige Anzeigeeinrichtung (3a, 3b, 3c) ein Bildschirm oder ein berührungsempfindlicher Bildschirm oder ein 3D-Bildschirm oder ein Projektor oder eine Datenbrille, bevorzugt eine Datenbrille für eine virtuelle und/oder erweiterte Realität, ist.

6. Verfahren nach einem der vorhergehenden Ansprüche,
**dadurch gekennzeichnet, dass**
das Verfahren automatisch bei einem Startvorgang des medizinischen bildgebenden Systems (1) durchgeführt wird.

7. Verfahren nach einem der vorhergehenden Ansprüche,
**dadurch gekennzeichnet, dass**
die Anwendungsprogramme (4a, 4b, 4c) zu unterschiedlichen Kategorien gehören, insbesondere jeweils zu einer der Kategorien Planung oder Bildgebung oder Nachbearbeitung oder Darstellung.

8. Verfahren nach einem der vorhergehenden Ansprüche,
**dadurch gekennzeichnet, dass**
die Anwendungsprogramme (4a, 4b, 4c) für die Verwendung in einem Ultraschallsystem und/oder in einem Mammographiesystem und/oder in einem Computertomographiesystem und/oder in einem Magnetresonanzsystem geeignet sind.

9. Verfahren nach einem der vorhergehenden Ansprüche,
**dadurch gekennzeichnet, dass**
für die Anwendungsprogramme (4a, 4b, 4c) in der Recheneinrichtung (2) zumindest ein Arbeitsablauf vorgegeben wird, durch welchen ein zeitlicher Ablauf für das Aktivieren der Anwendungsprogramme (4a, 4b, 4c) und ein Anzeigen einer jeweiligen Anwendungsprogramm-Information der jeweiligen Anwendungsprogramme (4a, 4b, 4c) auf der dem jeweiligen Anwendungsprogramm (4a, 4b, 4c) zugeordneten Anzeigeeinrichtung (3a, 3b, 3c) vorgegeben wird.

10. Verfahren nach Anspruch 9,
**dadurch gekennzeichnet, dass**
der Arbeitsablauf durch einen Benutzer (8) angepasst werden und der angepasste Arbeitsablauf in der Recheneinrichtung (2) hinterlegt werden kann.

11. Verfahren nach Anspruch 9 oder 10,
**dadurch gekennzeichnet, dass**
durch den Arbeitsablauf zumindest eine jeweilige Instanz der unterschiedlichen Anwendungsprogramme (4a, 4b, 4c) aktiviert wird, welche der Behandlung eines Patienten durch das medizinische bildgebende System (1) dient, und Instanzen, welche der Behandlung des selben Patienten dienen, als solche markiert und/oder gleichzeitig dargestellt und/oder auf benachbarten Anzeigebereichen (6a, 6a', 6b, 6b') und/oder nebeneinander angeordneten Anzeigeeinrichtungen (3a, 3b, 3c) angezeigt werden.

12. Medizinisches bildgebendes System (1) mit zumindest zwei unabhängig voneinander ausführbaren Anwendungsprogrammen (4a, 4b, 4c), mit
einer Recheneinrichtung (2), welche ausgebildet ist,
a) zumindest eine Anzeigeeinrichtung (3a, 3b, 3c) mit ihren für ein optisches Anzeigen relevanten Eigenschaften zu detektieren,
b) zumindest eine jeweilige anwendungsspezifische Anforderung der zumindest zwei unabhängig voneinander ausführbaren Anwendungsprogramme (4a, 4b, 4c) an eine Anzeigeeinrichtung (3a, 3b, 3c) für ein optisches Anzeigen von einer jeweiligen Anwendungsprogramm-Information auf einer Anzeigeeinrichtung (3a, 3b, 3c) abzurufen, wobei die abgerufene Anforderung einen oder mehrere der folgenden Parameter der jeweiligen Anzeigeeinrichtung (3a, 3b, 3c) umfasst: eine erforderliche oder empfohlene Bildauflösung, eine erforderliche oder empfohlene Bildwiederholrate, einen erforderlichen oder empfohlenen Farbraum, eine Kalibrierungsinformation, eine empfohlene oder erforderliche Größe einer Anzeigefläche (7a, 7b), eine empfohlene oder erforderliche Ausrichtung, eine Anordnungsinformation, eine Gattungsinformation, ob jeweils ein Bildschirm oder ein berührungsempfindlicher Bildschirm oder ein 3-D-Bildschirm oder ein Projektor oder eine Datenbrille empfohlen oder erforderlich ist;
c) die jeweils für die zumindest eine Anzeigeeinrichtung (3a, 3b, 3c) detektierten Eigenschaften mit der abgerufenen Anforderung zu vergleichen; und
d) in Abhängigkeit eines Ergebnisses des Vergleichens entweder die unabhängig voneinander ausführbaren Anwendungsprogramme (4a, 4b, 4c) jeweils zu der zumindest einen Anzeigeeinrichtung (3a, 3b, 3c) zuzuordnen oder eine Zuordnungsmöglichkeit für einen Benutzer (8) zu aktivieren.

## Claims

1. Method for operating a medical imaging system (1) having at least two independently executable application programs (4a, 4b, 4c), which method comprises the method steps:
a) a computing device (2) of the imaging system (1) detecting at least one display device (3a, 3b, 3c) by its properties relevant to visual display;
b) the computing device (2) retrieving at least one respective application-specific requirement of the at least two independently executable application programs (4a, 4b, 4c) on a display device (3a, 3b, 3c) for visual display of respective application program information on a display device (3a, 3b, 3c), wherein the retrieved requirement comprises one or more of the following parameters of the respective display device (3a, 3b, 3c):
a required or recommended image resolution; a required or recommended frame rate; a required or recommended colour space; calibration information; a recommended or required size of a display surface (7a, 7b), a recommended or required orientation; arrangement information; type information, which indicates whether a conventional screen or a touchscreen or a 3D screen or a projector or a pair of smartglasses is recommended or required in each case;
c) comparing the properties detected in each case for the at least one display device (3a, 3b, 3c) and the retrieved requirement;
d) according to a result of the comparison, the computing device (2) either assigning the independently executable application programs (4a, 4b, 4c) respectively to the at least one display device (3a, 3b, 3c) or activating an assignment facility for a user (8).

2. Method according to claim 1,
**characterised in that**
the assignment includes allocating different display areas (6a, 6a', 6b, 6b') of the at least one display device (3a, 3b, 3c) to the respective application programs (4a, 4b, 4c).

3. Method according to one of the preceding claims,
**characterised in that**
a plurality of display devices (3a, 3b, 3c) are detected, of which in particular at least two display devices (3a, 3b, 3c) differ in terms of their properties relevant to the visual display.

4. Method according to one of the preceding claims,
**characterised in that**
the at least one detected display device (3a, 3b, 3c), in particular one of the plurality of detected display devices (3a, 3b, 3c), is a screen or a touchscreen or a 3D screen or a projector or a pair of smartglasses, preferably a head pair of smartglasses for virtual and/or augmented reality.

5. Method according to one of the preceding claims,
**characterised in that**
the detected property comprises one or more of the following parameters of the respective display device (3a, 3b, 3c): a displayable image resolution; a displayable frame rate; a displayable colour space; calibration information; a size of a display surface (7a, 7b), an orientation of the display surface (7a, 7b); arrangement information; type information, which in particular indicates whether the respective display device (3a, 3b, 3c) is a screen or a touchscreen or a 3D screen or a projector or a pair of smartglasses, preferably a pair of smartglasses for virtual and/or augmented reality.

6. Method according to one of the preceding claims,
**characterised in that**
the method is performed automatically during a start-up procedure of the medical imaging system (1).

7. Method according to one of the preceding claims,
**characterised in that**
the application programs (4a, 4b, 4c) belong to different categories, in particular each belong to one of the categories planning or imaging or postprocessing or presentation.

8. Method according to one of the preceding claims,
**characterised in that**
the application programs (4a, 4b, 4c) are suitable for use in an ultrasound system and/or in a mammography system and/or in a computed tomography system and/or in a magnetic resonance system.

9. Method according to one of the preceding claims,
**characterised in that**
in the computing device (2) is defined for the application programs (4a, 4b, 4c) at least one workflow, which specifies a time sequence for activating the application programs (4a, 4b, 4c), and a display of respective application program information from the respective application programs (4a, 4b, 4c) on the display device (3a, 3b, 3c) assigned to the respective application program (4a, 4b, 4c).

10. Method according to claim 9,
**characterised in that**
the workflow can be adapted by a user (8), and the adapted workflow can be stored in the computing device (2) .

11. Method according to claim 9 or 10,
**characterised in that**
the workflow activates at least one respective instance of the various application programs (4a, 4b, 4c), which is used to treat a patient by the medical imaging system (1), and instances that are used to treat the same patient are labelled as such and/or displayed simultaneously and/or displayed on adjacent display areas (6a, 6a', 6b, 6b') and/or adjacently arranged display devices (3a, 3b, 3c).

12. Medical imaging system (1) having at least two independently executable application programs (4a, 4b, 4c), with a computing device (2) that is designed to:
a) detect at least one display device (3a, 3b, 3c) by its properties relevant to visual display;
b) retrieve at least one respective application-specific requirement of the at least two independently executable application programs (4a, 4b, 4c) on a display device (3a, 3b, 3c) for visual display of respective application program information on a display device (3a, 3b, 3c), wherein the retrieved requirement comprises one or more of the following parameters of the respective display device (3a, 3b, 3c): a required or recommended image resolution; a required or recommended frame rate; a required or recommended colour space; calibration information; a recommended or required size of a display surface (7a, 7b), a recommended or required orientation; arrangement information; type information, which indicates whether a conventional screen or a touchscreen or a 3D screen or a projector or a pair of smartglasses is recommended or required in each case;
c) compare the properties detected in each case for the at least one display device (3a, 3b, 3c) with the retrieved requirement; and
d) according to a result of the comparison, either assign the independently executable application programs (4a, 4b, 4c) respectively to the at least one display device (3a, 3b, 3c) or activate an assignment facility for a user (8).

## Revendications

1. Procédé pour faire fonctionner un système (1) d'imagerie médicale par au moins deux programmes (4a, 4b, 4c) d'application, pouvant être exécutés indépendamment les uns des autres, comprenant les stades de procédure :
a) détection par un dispositif (2) informatique du système (1) d'imagerie d'au moins un dispositif (3a, 3b, 3c) d'affichage avec ses propriétés pertinentes pour un affichage optique ;
b) appel, par le dispositif (2) informatique, d'au moins une demande respective spécifique à une application des au moins deux programmes (4a, 4b, 4c) d'application, pouvant être exécutés indépendamment les uns des autres, sur un dispositif (3a, 3b, 3c) d'affichage pour un affichage optique sur un dispositif (3a, 3b, 3c) d'affichage d'une information respective de programme d'application, la demande appelée comprenant un ou plusieurs des paramètres suivants du dispositif (3a, 3b, 3c) d'affichage respectif : une résolution d'image nécessaire ou ressentie, un taux de rafraîchissement d'image nécessaire ou ressenti, un espace chromatique nécessaire ou ressenti, une information d'étalonnage, une dimension ressentie ou nécessaire d'une surface (7a, 7b) d'affichage, une orientation ressentie ou nécessaire, une information d'agencement, une information de type, sur le point de savoir si, respectivement, un écran ou un écran tactile ou un écran en 3D ou un projecteur ou des lunettes de données est ressenti ou nécessaire ;
c) comparaison de la demande appelée et des propriétés détectées, respectivement, du au moins un dispositif (3a, 3b, 3c) d'affichage ;
d) en fonction d'un résultat de la comparaison, soit une association respective des programmes (4a, 4b, 4c) d'application, pouvant être exécutés indépendamment les uns des autres, au au moins un dispositif (3a, 3b, 3c) d'affichage, soit une activation d'une possibilité d'association pour un utilisateur (8) par le dispositif (2) informatique.

2. Procédé suivant la revendication 1,
**caractérisé en ce que**
l'association comprend une attribution de régions (6a, 6a', 6b, 6b') d'affichage différentes du au moins un dispositif (3a, 3b, 3c) d'affichage aux programmes (4a, 4b, 4c) d'application respectifs.

3. Procédé suivant l'une des revendications précédentes,
**caractérisé en ce que**
l'on détecte plusieurs dispositifs (3a, 3b, 3c) d'affichage, dont notamment au moins deux dispositifs (3a, 3b, 3c) d'affichage se distinguent par leurs propriétés pertinentes pour l'affichage optique.

4. Procédé suivant l'une des revendications précédentes,
**caractérisé en ce que**
le au moins un dispositif (3a, 3b, 3c) d'affichage détecté, notamment l'un des plusieurs dispositifs (3a, 3b, 3c) d'affichage détectés, est un écran ou un écran tactile ou un écran en 3D ou un projecteur ou une lunette de données, de préférence une lunette de données pour une réalité virtuelle et/ou augmentée.

5. Procédé suivant l'une des revendications précédentes,
**caractérisé en ce que**
la propriété détectée comprend un ou plusieurs des paramètres suivants du dispositif (3a, 3b, 3c) d'affichage respectif : une résolution d'image pouvant être représentée, un taux de rafraîchissement d'image pouvant être représenté, un espace chromatique pouvant être représenté, une information d'étalonnage, une dimension d'une surface (7a, 7b) d'affichage, une orientation de la surface (7a, 7b) d'affichage, une information d'agencement, une information de type, dont il ressort notamment que le dispositif (3a, 3b, 3c) respectif d'affichage est un écran ou un écran tactile ou un écran en 3D ou un projecteur ou une lunette de données, de préférence une lunette de données pour une réalité virtuelle et/ou augmentée.

6. Procédé suivant l'une des revendications précédentes,
**caractérisé en ce que**
l'on effectue le procédé automatiquement lors d'une opération de lancement du système (1) d'imagerie médicale.

7. Procédé suivant l'une des revendications précédentes,
**caractérisé en ce que**
les programmes (4a, 4b, 4c) d'application appartiennent à des catégories différentes, notamment respectivement à l'une des catégories planification ou imagerie ou post-traitement ou représentation.

8. Procédé suivant l'une des revendications précédentes,
**caractérisé en ce que**
les programmes (4a, 4b, 4c) d'application sont appropriés à l'utilisation dans un système à ultrasons et/ou dans un système de mammographie et/ou dans un système de tomographie assisté par ordinateur et/ou dans un système à résonnance magnétique.

9. Procédé suivant l'une des revendications précédentes,
**caractérisé en ce que**,
pour les programmes (4a, 4b, 4c) d'application, on prescrit, dans le dispositif (2) informatique, au moins un déroulement des opérations, par lequel on prescrit un déroulement en fonction du temps de l'activation des programmes (4a, 4b, 4c) d'application et un affichage d'une information respective de programme d'application des programmes (4a, 4b, 4c) d'application respectifs sur le dispositif (3a, 3b, 3c) d'affichage associé au programme (4a, 4b, 4c) d'application respectif.

10. Procédé suivant la revendication 9,
**caractérisé en ce que**
le déroulement des opérations est adapté par un utilisateur (8) et le déroulement adapté des opérations peut être mémorisé dans le dispositif (2) informatique.

11. Procédé suivant la revendication 9 ou 10,
**caractérisé en ce que**
l'on active, par le déroulement des opérations, au moins une instance respective des programmes (4a, 4b, 4c) d'application différents, qui sert à traiter un patient par le système (1) d'imagerie médicale, et des instances, qui servent à traiter le même patient, sont repérées en tant que telles et/ou représentées en même temps et/ou affichées sur des régions (6a, 6a', 6b, 6b') d'affichage voisines et/ou des dispositifs (3a, 3b, 3c) d'affichage disposés les uns à côté des autres.

12. Système (1) d'imagerie médicale ayant au moins deux programmes (4a, 4b, 4c) d'application, pouvant être exécutés indépendamment les uns des autres, comprenant un dispositif (2) informatique, constitué pour
a) détecter au moins un dispositif (3a, 3b, 3c) d'affichage avec ses propriétés pertinentes pour un affichage optique ;
b) appeler au moins une demande respective spécifique à une application des au moins deux programmes (4a, 4b, 4c) d'application, pouvant être exécutés indépendamment les uns des autres, sur un dispositif (3a, 3b, 3c) d'affichage pour un affichage optique d'une information de programme d'affichage respective sur un dispositif (3a, 3b, 3c) d'affichage, la demande appelée comprenant un ou plusieurs des paramètres suivants du dispositif (3a, 3b, 3c) d'affichage respectif : une résolution d'image nécessaire ou ressentie, un taux de rafraichissement d'image nécessaire ou ressenti, un espace chromatique nécessaire ou ressenti, une information d'étalonnage, une dimension ressentie ou nécessaire d'une surface (7a, 7b) d'affichage, une orientation ressentie ou nécessaire, une information d'agencement, une information de type, sur le point de savoir si, respectivement, un écran ou un écran tactile ou un écran en 3D ou un projecteur ou des lunettes de données est ressenti ou nécessaire ;
c) comparer les propriétés détectées, respectivement, pour le au moins un dispositif (3a, 3b, 3c) d'affichage à la demande appelée ; et
d) en fonction d'un résultat de la comparaison, soit associer les programmes (4a, 4b, 4c) d'application, pouvant être exécutés indépendamment les uns des autres, respectivement, au au moins un dispositif (3a, 3b, 3c) d'affichage, soit activer une possibilité d'association pour un utilisateur (8).
